# EUROPEAN PATENT SPECIFICATION

(11) **EP 2 403 509 B1**
(45) Date of publication and mention of the grant of the patent: **21.08.2013**
(21) Application number: 10723285.2
(22) Date of filing: 05.03.2010
(51) Int. Cl.: A61K 35/14, A61P 17/02

(54) **ACTIVATED LEUKOCYTE COMPOSITION**
AKTIVIERTE LEUKOZYTEN-ZUSAMMENSETZUNG
COMPOSITION DE LEUCOCYTES ACTIVÉS

(30) Priority: 05.03.2009 US 209298 P; 01.04.2009 US 211587 P
(43) Date of publication of application: 11.01.2012
(60) Divisional application: 13176068.8
(73) Proprietor: Macrocure, Ltd., 49250 Petach Tikva (IL)
(72) Inventor: SHIRVAN, Mitchell, 46432 Herzliyya (IL); SHINAR, Eilat, Jerusalem (IL); FRENKEL, Orit, 52621 Tel Hashomer (IL); ZULOFF-SHANI, Adi, 46432 Herzliyya (IL); BUBIS, Marina, 76303 Rehovot (IL); BAIN, Eilat, 76303 Rehovot (IL); GINIS, Irene, 99557 Beit Shemesh (IL)
(74) Representative: Krishnan, Sri
(86) International application number: PCT/IB2010/000882
(87) International publication number: WO 2010/100570

(56) References cited:
- US-A- 6 146 890

## Description

### CROSS-REFERENCE TO RELATED APPLICATIONS

The present application is related to Application Serial No. 61/209,298, filed March 5, 2009, entitled Activated Leukocyte Composition, and Application Serial No. 61/211,587, filed April 1, 2009, entitled Activated Leukocyte Composition for Vascular Insufficiencies.

### BACKGROUND OF THE INVENTION

The wound healing process involves participation of white blood cells, also known as leukocytes. Leukocytes include lymphocytes, granulocytes and monocytes. Three common types of lymphocytes are T-cells, B-cells and natural killer cells. T-cells and B-cells play important roles in the recognition of antigens in the body (Parkin, 2001). Natural killer (NK) cells identify infected cells by alterations in the levels of the major histocompatability complex (MHC), and destroy the infected cells (Moretta, 2008). The participation of lymphocytes in the healing process is largely associated with their production of cytokines and growth factors (Keen, 2008). A new class of gamma-delta-T cells has been described in the skin (Jameson, 2002. Havran, 2005). Among the different types of granulocytes are neutrophils, basophils and eosinophils. Monocytes differentiate into macrophages, which are responsible for destruction of tissue debris or invading foreign substances. Macrophages also produce molecules that control inflammation and repair (Riches, 1996).

The process of wound healing occurs in three overlapping phases. (Li, 2007; Broughton, 2006; Tsirogianni, 2006; Singer, 1999; Martin, 1997). The first phase is the inflammatory phase. It is characterized by recruitment of neutrophils, followed by monocytes to the wound site, where they kill and phagocytize bacteria (Agaiby, 1999).

The second wound healing phase which is known as the proliferative phase, involves formation of new granulation tissue. Fibroblasts proliferate and migrate into the wound space and synthesize collagen and other components of extracellular matrix (Greiling, 1997). At the same time, angiogenesis occurs, providing nutrients and oxygen to the metabolically active new granulation tissue (Tonnesen, 2000). Keratinocytes from the intact epidermis start to migrate over the provisional matrix and begin to proliferate, leading the way for new epithelial tissue (Kim, 1992).

Remodeling is the third and final phase in wound healing. It is characterized by fibroblast differentiation into myofibroblasts, which contract and bring the wound edges closer together (Tomasek, 2002). Remodeling of the collagen fibers by degradation and re-synthesis allows the wound to gain strength by re-orientation of the collagen fibers (a process tightly controlled by growth factors) (Werner, 2003).

The challenge of treating wounds is often compounded by patients with multiple pathologies such as diabetes, coronary artery disease and hypertension. These diseases have the common effect of exacerbating vascular complications due to various physiological conditions. Complications from wounds may result in increased morbidity and mortality (Doshi, 2008).

Conventional wound treatments include surgical debridement, antibiotic therapies and various dressings (Moran, 2008; Fonder, 2008). Wounds resistant to conventional treatment are also referred to as refractory wounds. These wounds lead to a decrease in quality of life and can result in increased morbidity and mortality. Thus, a need continues to exist for effective wound healing compositions and methods.

### SUMMARY OF THE INVENTION

The present invention relates to a method for making an activated leukocyte composition comprising:
a. incubating human leukocytes at a (i) temperature of 12° C - 28°C for a time ranging from about 90 minutes upwards of about 2, 3, 4, 5, 6, 7, 8, or 12 to 20 hours or (ii) temperature of 12°C - 37°C for a time ranging from 5-24 hours so that the leukocytes transition from a quiescent to a functionally active state;
b. subjecting the leukocytes to hypo-osmotic shock; and
c. adding to the leukocytes of step b, a physiologically acceptable salt solution in an amount effective to restore isotonicity.
One aspect of the present invention is directed to a method for making an activated leukocyte composition (ALC) derived from blood (e.g., obtainable or obtained from a whole blood sample) The method includes the steps of subjecting leukocytes, which may be obtained from a sample of whole human blood, to a first incubation for a period of time and at a temperature which allows the leukocytes to become activated, which in preferred embodiments, is about 8 to about 20 hours, and at room temperature. After incubation, the leukocytes are contacted with a physiologically acceptable aqueous solution such as sterile, distilled water, to initiate hypo-osmotic shock, followed by contacting the shocked leukocytes with a physiologically acceptable salt solution to restore isotonicity. This activated leukocyte composition (ALC) may be used therapeutically. However, in some embodiments, separate and substantially concurrent with the first incubation of the leukocytes, a sample of plasma, which may be obtained from the same or different whole blood sample (i.e., from the same or a different human), is contacted with a coagulating agent at about 37° C concurrent with the leukocyte incubation, which in preferred embodiments, is about 8 to about 20 hours, followed by separating serum from the coagulated plasma sample. The leukocytes are re-suspended in serum collected from the coagulated plasma sample, thus forming the ALC. After the first incubation, the leukocytes may be further subjected to a second incubation for about 60 to about 120 minutes at about 37°C.

Another aspect of the present invention is directed to an ALC derived from blood. The activated leukocyte composition of the present invention includes, in terms of the population of leukocytes present therein, about 40% to about 90% granulocytes, about 5% to about 20% monocytes and about 5% to about 30% lymphocytes, based on the total number of leukocytes in the ALC. As shown in the working examples, the inventive ALCs may also be characterized and distinguished from known compositions in terms of minimum yield of leukocytes (relative to the whole blood sample), viability of leukocytes, and minimum activation levels of granulocytes, *e.g*., as indicated by CD11b. The ALC may further contain residual levels of platelets (in amounts of about 46.8+/- 39.2(10³/µl) and red blood cells (in the amount of about 0.1 +/-0.06(10⁶/µl) of the ALC. The population of lymphocytes may include about 7% to about 25% B cells (CD19+), about 20% to about 30% NK cells (CD3-/CD56+), about 40% to about 60% T cells (CD3+), about 0.1% to about 30% of NKT cells CD3+/CD56+, about 8% to about 20% of T helper cells (CD4+/CD3+), and about 20% to about 30% of CD8+/CD3+ cells. The cells may be suspended in a carrier such as serum (which may be autologous or allogeneic with respect to recipient) or some other physiologically acceptable iso-normal liquid suitable for storing and administering cells, such as the solution used to restore isotonicity.

The present invention can be used for promoting wound healing, by administering or otherwise applying the ALC to a wound.

The disclosed invention achieves several unexpected results compared to at least one known wound healing composition containing white blood cells. As demonstrated in working examples herein, these results include increased yield and viability of leukocytes (WBCs), and higher percentage of activated granulocytes. The disclosed invention is also believed to include an unexpectedly higher percentage of activated monocytes and a relatively higher percentage of CD4 T-cells compared to CD8 T-cells.

### BRIEF DESCRIPTION OF THE DRAWINGS

Figure 1 schematically depicts a first portion of a representative system for producing the ALC compositions of the present invention, which includes bags A-C (Set-1), which are blood storage bags or containers, wherein bag A contains packed red blood cells collected from a donor; bag B contains plasma; and bag C contains leukocytes (which following initial separation from whole blood forms a layer commonly referred to as the buffy coat).

Figure 2 schematically depicts a second portion of the representative system for producing ALC compositions of the present invention, which includes a 7-bag set after bag A with RBC is removed from the system and bags B and C from figure 1 are welded to bags 1-5 (Set-2).

Figures 3A and 3B are graphs illustrating the general trends of both CD62L and CD42b expression, which are indicators of cell activation.

### DETAILED DESCRIPTION

Blood is defined herein as whole blood or any of its constituent parts (*e.g*., plasma, leukocytes, platelets or red blood cells). The amounts of platelets and red blood cells that may be present in the ALC of the present invention may be lower than that in whole blood.

The term "about" as used herein in connection with any and all values (including lower and upper ends of numerical ranges) as any value having an acceptable range of deviation of +/- 0.5% to +/- 20% (and values therebetween, *e.g*., ± 1%, ± 1.5%, ± 2%, ± 2.5%, ± 3%, ± 3.5%, ± 4%, ± 4.5%, ± 5%, ± 5.5%, ± 6%, ± 6.5%, ± 7%, ± 7.5%, ± 8%, ± 8.5%, ± 9%, ± 9.5%, ± 10%, ± 10.5 %, ± 11%, ± 11.5%, ± 12%, ± 12.5%, ± 13, ± 13.5%, ± 14%, ± 14.5%, ± 15%, ± 15.5%, ± 16%, ± 16.5%, ± 17%, ± 17.5%, ± 18%, ± 18.5%, ± 19%, ± 19.5%, and ± 20%).

The starting materials for producing the inventive ALCs may be obtained from several sources. Whole blood or one or more components thereof (*e.g*., leukocytes and plasma) may be obtained from autologous or allogeneic sources. In one embodiment of the present invention, the blood sample is collected from the patient who will ultimately be treated with the ALC, which is referred to herein as an autologous blood sample or source. In embodiments wherein the source(s) i.e., the blood or its components, is obtained from an individual other than the intended ALC recipient, which is referred to as an allogeneic blood sample or source, these starting materials may be conveniently obtained from a blood bank. The samples may be screened by the blood bank for blood type (ABO, Rh), irregular antibodies to red cell antigens, and transfusion-transmittable diseases. More specifically, screening can be conducted with antibodies using an Abbott PRISM instrument against: Hepatitis B, C, HIV 1/2, HTLV and Syphilis (-HCV; HbsAg; anti-HIV 1/2 O+; and anti-HTLV I/II). The samples can also be screened for HIV, HCV and HBV by molecular methods (NAT-Nucleic Acid Testing). Molecular screening can be accomplished using commercially available instrumentation, e.g., the TIGRIS system of Chiron.

In these embodiments involving allogeneic sources, the samples can be obtained from donors with the same blood type as the intended ALC recipient. Alternatively and as further described herein, plasma samples can be obtained from donors with AB+ blood and the leukocytes can be obtained from patients with O- blood. Patients with AB+ blood are universal donors for plasma and patients with O- blood are universal donors for leukocytes. Regardless of the source, all necessary processing of the sample(s) can be carried out without the need for highly specialized equipment.

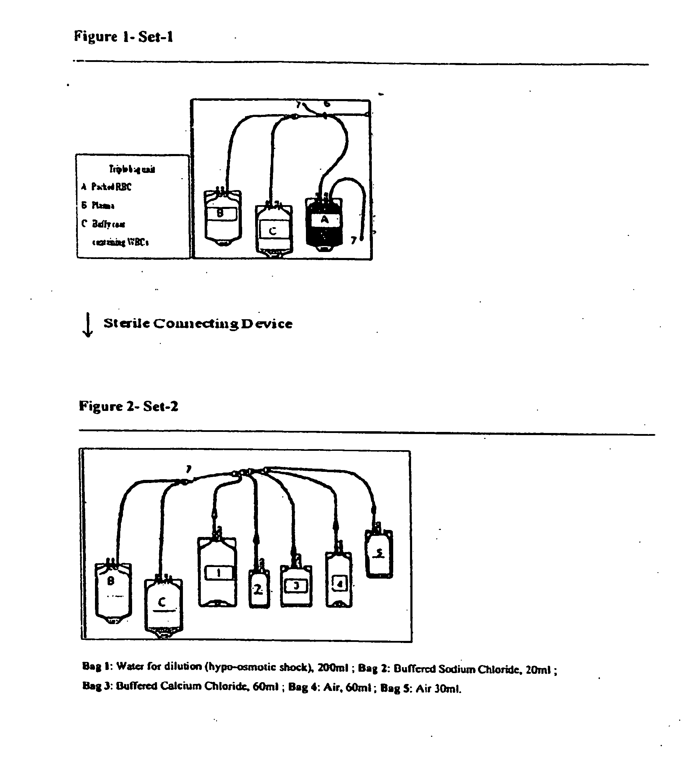

A preferred method of making the ALC composition of the present invention is now described with reference to Figs. 1 and 2, which illustrate a system containing two sets of interconnected sterile infusion bags. The system is sealed so that there is no exposure to the outside environment. Specifically, the tubes connecting the two sets are welded together to form one system using a Sterile Connecting Device (*e.g*., TSCD®-II Cat number ME-203AH of Terumo). More specifically, to ensure compliance with sterility standards, the welding and cutting of the tubes is done by pre-heating special wafers to about 300°C. This high temperature increases the sterility of the welding procedure. To further ensure sterility, the welding may be performed in a class 100 Biological Safety Cabinet within a class 100,000 containment area.

As illustrated in these figures, the system contains two sterile bag sets. Set 1, containing bags A, B, and C, is a standard, commercially available triple bag set commonly used for blood transfusion. A human blood sample, typically in the volume of about 400 to about 550 ml, is collected in a blood bank via venipuncture and placed into bag A, and then fractionated into its component parts using standard techniques into bags A, B and C. For example, bag A containing the blood sample is centrifuged. After centrifugation, the blood components are separated, *e.g*., using a blood component extractor manufactured by Baxter. The buffy coat containing leukocytes is placed into bag C, plasma is placed into bag B and erythrocytes remain in bag A. Thus, as a result of this process; bag A contains packed erythrocytes; bag B contains plasma; and bag C contains the buffy coat containing leukocytes (and possibly residual plasma and erythrocytes). Alternatively, the blood components can be separated from whole blood via apheresis techniques known in the art.

Bag A is then disconnected from the three-bag set. As illustrated in Fig. 2, bags B and C are then welded to custom made infusion bags 1-5 (Set-2) to form the system used to make the activated leukocyte composition. As disclosed above, welding is performed with a sterile connecting device. Bag 1 contains a first aqueous solution (*e.g*., 200ml of sterile distilled water), which is used for the purpose of exposing the cells in the buffy coat to hypo-osmotic shock. This serves to lyse residual erythrocytes that may be present. Bag 2 contains a second solution (*e.g*., 20ml of buffered sodium chloride solution (8.91% NaCl, USP), or any other other physiologically acceptable solution containing inorganic ions, organic osmolytes such as sucrose, or some combination thereof, such as Lactated Ringers (Hartmans) solution), which serves to restore the leukocytes to isotonicity following hypo-osmotic shock. When the sodium chloride solution is added to 200ml of distilled water, it becomes a 0.9% NaCl solution. Bag 3 contains a third solution (*e.g*., about 60ml of buffered calcium chloride solution (1.17% CaCl₂ Dihydrate, USP)), which acts to coagulate the plasma in bag B, and to facilitate separation into platelets and serum. Bag 4 and bag 5 each contains sterile filtered air, about 60ml and about 500ml respectively. The set is packed as a single unit and sterilized using high pressure steam, which greatly reduces the risk of secondary infection to the patient.

The leukocytes are then transferred from bag C into bag 4 (or bag 5) and incubated, preferably in a vertical position, to allow them to activate. For purposes of the present invention, cell activation is defined as a process by which the cells (leukocytes) are allowed to become activated, and more specifically, as a transition from a quiescent to a functionally active state which is accompanied by synthesis of biologically active substances or translocation of pre-synthesized substances from cytoplasm to the cellular membrane or their release out of the cells. These substances may include proteins or polypeptides, lipids, sugars, oxygen radicals and other biochemical moieties that function as adhesion molecules, cytokines, growth factors, enzymes, transcription factors and cell signaling receptors and mediators. When detected and identified inside the cells or on the cell surface, these molecules are called activation markers.

In some embodiments, the leukocytes are incubated simply by allowing them to stand at room temperature. For purposes of the present invention, room temperature refers to a temperature in the range of about 12°C to about 28°C, and in some embodiments from about 16°C to about 25°C. The time period of incubation may vary depending upon the temperature. Incubation times will be lower at increased temperatures. The incubation time needed to activate the leukocytes will be roughly inversely proportional to the temperature at which the incubation is conducted. For example, in embodiments where leukocytes are allowed to stand at room temperature, the incubation time generally ranges from about 90 minutes, and upwards of 2, 3, 4, 5, 6, 7, or 8 to about 20 hours. In preferred embodiments, the incubation time ranges from about 8 to about 20 hours. In a more preferred embodiment, incubation of the leukocytes occurs at about 18°C to about 24°C for about 8 hours to about 12 hours. In other embodiments, incubation of the leukocytes involves exposing them to heat, e.g., at a temperature above room temperature and up to about 37°C. The time period for incubation at elevated temperatures generally ranges from 5 hours to about 24 hours.

Referring again to Fig. 2, the leukocytes in bag C are transferred to bag 4 which is typically smaller than bag C (*e.g.* about 250ml - about 500ml). Also, cells can be transferred into another 500ml bag (bag 5). To allow for activation of the leukocytes, bag 4 (or bag 5) is placed in a vertical position and incubated, for about 8 to about 20 hours at room temperature.

After incubation, the leukocytes are subjected to hypo-osmotic shock, which lyses erythrocytes. In preferred embodiments, hypo-osmotic shock is performed immediately (*i.e*., upon completion of the preceding step without any intervening step or unnecessary delay, typically less than 2 minutes). The hypo-osmotic shock may be initiated by transferring the distilled water from bag 1 to bag 4 (or bag 5) containing the leukocytes. The hypo-osmotic shock treatment is typically conducted for about 45 seconds. Following this step, and preferably immediately thereafter, isotonicity is restored to the leukocytes by transferring the sodium chloride solution from bag 2 to bag 4 (or bag 5). The ratio of the volume of sodium chloride solution to cell suspension in water is generally about 1:10. The contents of bag 4 are then transferred into Bag 1 (or left in bag 5), which is larger in volume than bag 4. As described above, this composition may be used therapeutically in the inventive methods.

The preferred embodiment involves at least one additional step. Thus, in a separate step, which may be conducted concurrently with the leukocyte incubation, plasma is separated into platelets and serum by the use of a coagulant such as CaCl₂, followed by centrifugation Thus, in this representative embodiment, CaCl₂ from bag 3 is transferred to bag B. Bag B, which now contains a composition of plasma and CaCl₂. is typically allowed to coagulate at a temperature of about 37°C. The plasma remains in contact with the coagulating agent for substantially the same period of time the leukocytes are incubated.

After hypo-osmotic shock and restoration of isotonicity of the leukocytes, and subsequent transfer of the contents of bag 4 into bag 1 (or remain in bag 5, and after coagulation of plasma in bag B, the entire 7-bag system is centrifuged. In preferred embodiments, the centrifugation is conducted immediately following transfer of the leukocytes into bag 1 (or remain in bag 5), in order not to expose cells to hemolysate After centrifugation, the supernatant from bag 1 (or bag 5) is transferred into bag C, and the leukocyte pellet formed in bag 1 (or bag 5) as a result of the centrifugation is re-suspended in about 20ml to about 50ml of serum from bag B.

In yet another step of the preferred embodiment of the present invention, the activated leukocytes may be incubated in the coagulated plasma mentioned above before the final composition is made. Generally, this second incubation period is conducted for about 1-2 hours at 37°C.

In other embodiments, the ALC composition may be prepared from smaller volumes of blood samples, with commensurate decreases in volumes of all solutions and use of smaller bags. Furthermore, use of these different size bags yield ALCs with different compositions. Even in these embodiments, allogeneic or autologous blood samples may be used as starting materials. Use of smaller volumes provides the clinician with the ability to perform the blood collection autonomously, without using an external blood bank, such as in emergency situations in treating patients with otherwise healthy immune systems but suffering from some type of traumatic wound (*e.g*., battlefield and combat conditions). In these embodiments, testing for transmittable diseases and antigens may be dispensed with. However in such cases, patients with refractory wounds are not clinically acceptable blood donors for effective ALC preparation. When this situation arises, ALC will be produced from allogeneic donors by the means described herein.

The ALCs of the present invention include leukocytes, *e.g*., granulocytes, monocytes and lymphocytes. Granulocytes include neutrofils, eosinophils and basofils. In its broadest sense, the leukocyte population of the ALC generally contains about 40% to about 90% granulocytes, about 5% to about 20% monocytes and about 5% to about 30% lymphocytes. Specific amounts of the cells may differ based on the analysis techniques employed. When analysis is performed using FACS (*e.g*., using a side-scatter versus a forward-scatter dot plot analysis), the leukocyte composition generally contains about 55% to about 80% granulocytes; about 5% to about 15% monocytes and about 5% to about 30% lymphocytes, and in some embodiments, comprises about 58-76% granulocytes; about 5-11% monocytes and about 9-23% lymphocytes. When analysis is performed using a Cell Dyn Analyzer, the leukocyte composition generally contains about 50% to about 90% granulocytes; about 5% to about 15% monocytes; and about 10% to about 25% lymphocytes. The subpopulation of lymphocytes in the ALC may confirm the following cells in the general ranges as follows: about 7% to about 25% B cells (CD19+); about 20% to about 30% NK cells (CD3-/CD56+), about 40% to about 60% T cells (CD3+); about 0.1% to about 30% of NKT cells CD3+/CD56+, about 8% to about 20% of T helper cells (CD4+/CD3+), and about 20% to about 30% of CD8+/CD3+ cells. In preferred embodiments, the lymphocyte subpopulation is enriched with at least 9% CD56+ cells (CD3-/CD56+;CD3+/CD56+;CD3+/CD56+/CD8+), the amount of the T helper lymphocytes (CD4+/CD3+) is decreased to less than 20%, and/or the ratio of T-helper to T-suppressor cells (CD4+/CD3+:CD8+/CD3+) is less than 0.8.

Patients suffering from wounds can be physiologically compromised or otherwise healthy. For example, due to already impaired metabolic systems, diabetics and other medically compromised patients are candidates for ALC derived from heterologous blood, as their own leukocytes may not be optimal for the procedure. However, otherwise healthy patients, as in the example of trauma patients, are also good candidates for ALC compositions of the present invention.

The present invention is useful in promoting healing in a multitude of wound types. Although in practice it may be used in combination with other treatment modalities, it does not require them to achieve effective wound healing. The inventors have contemplated application of the ALC to any type of wound and foresee no limitations as to the type of wound that can be treated. The ease of application, e.g., with a standard syringe or similar application device, makes the inventive ALC compositions safe and easy to use.

Wounds amenable to treatment with the invention are typically in the form of punctures, cuts or tears of the living tissues. Wounds of the skin can penetrate the epidermis, dermis or in the case of full-thickness wounds, the subcutaneous tissue. Thus, representative types of wounds amenable to treatment with the compositions and methods of the present invention include decubital or pressure ulcers; diabetic ulcers, deep sternal wounds, *e.g*., following open heart surgery (to the great saphenous vein after coronary revascularization and harvesting of the great saphenous vein); and post-operative wounds following abdominal and any other types of surgery. Other wounds are those which result from trauma such as by gun shots, knives, or any other object able to cause a cut or tear in the skin. Wounds of the oral cavity (*e.g*., teeth), as well as wounds that arise as a side-effect of medication or as a symptom of various pathologies (*e.g*., sores associated with Kaposi's Sarcoma), as well as internal wounds (*e.g*. anal fissures, and wounds or lesions to the gastrointestinal tract, such as ulcers in the stomach or intestines) may also be amenable to treatment with the present invention.

The ALC may also be used to treat any wounds exacerbated by vascular insufficiency. Vascular insufficiency, for purposes of the present invention, refers to inadequate blood circulation resulting in insufficient perfusion to the afflicted areas. Such insufficiency can be caused by trauma (*e.g*. damage to the vasculature adjacent to a skeletal fracture), or various pathologies (*e.g*. diabetes and atherosclerosis). In either instance, whether trauma or disease induced, vascular insufficiency decreases the likelihood of effective wound healing. The ALC may be useful in improving wound healing outcomes in these patients and should be administered according to the methods described herein. Additionally, treatment algorithms should not be limited by the severity or type of wound, or the extent of vascular insufficiency. ALC may be more efficacious in patients presenting with the most severe wounds and vascular insufficiency.

In general, application of the activated leukocyte composition is accomplished by means of one or more injections of the ALC directly into the wound or the tissue surrounding the wound. The ALC may be applied directly into an open wound.

For injection into the wound, it is preferred to use a Luer-Lock syringe or any other commercially available syringe that has a locking mechanism between the syringe and the needle. The biological space of a wound, particularly a pressure wound, is often limited. When injecting into a wound, there is a risk of pressure causing the syringe to separate from the needle. Using a locking syringe eliminates this risk.

When injection into the wound tissue is not possible, the ALC can be applied directly into the cavity of the wound. Application in this method can be done using direct application with a syringe or tubing.

The ALC may be applied to or around the wound site with the aid of a dressing. Dry dressings include gauze and bandages, non-adhesive meshes, membranes and foils, foams, and tissue adhesives. Moisture-keeping barrier dressings include pastes, creams and ointments, nonpermeable or semi-permeable membranes or foils, hydrocolloids, hydrogels, and combination products. Bioactive dressings include antimicrobial dressings, interactive dressings, single-component biologic dressings, and combination products. In some embodiments, the wound is packed with sterile gauze soaked in the ALC. The dressing, e.g., such as sterile gauze pads, may be saturated with compositions such as Lactated Ringer (Hartman) Solution, alginate containing dressing, polyurethane dressing or carboxymethylcellulose dressing, which is applied to cover the wound, followed by application of dry dressing. If the subject wound is highly infected, then silver dressings such as Silverlon can be applied. The choice of post-injection dressing is based on the determination of the clinician. Commercial availability, history of past clinical success, and patient tolerance are all factors to be considered in the selection of a wound dressing. The dressing may be removed periodically, *e.g*., typically after about 24 hours, in order to irrigate the wound *e.g*., with sterile water and soap.

In another embodiment, the composition can be placed onto a physiologically inert and/or resorbable matrix or scaffold (*e.g*., collagen) and inserted by means of a press fit, into the wound. This allows for a sustained delivery of the ALC into the site which benefits the patient in that the cells have a longer period *in situ.*

The ALC compositions may be applied to the wound once or more than once, *e.g.*, after 4 weeks, once a clinician determines whether another application is necessary. Factors that may be taken into account include increased wound dimensions (width, length and depth), suppuration, pyrexia or any other sign or symptom indicating a recalcitrant infection such that re-treatment is warrranted. In addition to re-treatment, referral for surgical debridement may be indicated at any point the clinician deems appropriate.

The ALC may be used in conjunction with any other conventional wound treatment, such as warming (therapeutic heat), electrical stimulation, magnetism, laser phototherapy, cycloidal vibration therapy and ultrasound. It also can be used with biological therapy such as larva therapy, skin substitutes, cultured keratinocytes (Epicel, Genzyme biosurgery), human dermal replacement (Dermagraft, Smith and Nephew Inc.), cadaver derived processed dermis (Alloderm, Life Cell Corporation), Bilayered Skin Equivalent (Apligraf, Organogenesis Inc.), TransCyte (Smith and Nephew Inc.), Growth Factors (PDGF is currently the only growth factor licensed for topical use), and fibrin sealant. In some embodiments, the ALC is used in conjunction with VAC, which is a commercially available wound therapy manufactured by KCI. VAC promotes wound healing by applying negative pressure to a wound. In these embodiments, ALC is preferably applied to a wound prior to VAC therapy. In yet other embodiments, the ALC is used in conjunction with hyperbaric therapy (Thackham, 2008). For example, the ALC can be applied to a wound just prior to a patient receiving hyperbaric therapy. The ALC may also be used in conjunction with low-energy shock wave therapy (*e.g*., impulses of about 0.1 mJ/mm²; 5 Hz) per centimeter of wound length). *See, e.g*., Dumfarth, et al., Ann. Thorac. Surg. 86:1909-13 (2008).

After treatment, the wounds may be evaluated for length, width and height measurements. Typically, a wound is considered healed when all measurements of these parameters are negligible. The ALC may also provide an analgesic effect.

The activated leukocyte composition is particularly useful in wounds including diabetic foot ulcers and decubital ulcers. Decubital ulcers are pressure ulcers caused by impeded blood flow, usually due to prolonged pressure on a particular area. (Berlowitz, 2007) Decubital ulcers cause morbidity and mortality in elderly people. At least 48% of stage IV pressure ulcers remain unhealed after one year of treatment. (Girouard, 2008). Patients suffering from decubiti also commonly have co-morbid pathologies such as diabetes and hypertension. These pathologies further complicate the successful treatment of decubiti.

For treating decubital ulcers, the composition is aspirated into a sterile syringe of any size, using an 18-gauge (18G) needle. Aspiration is performed slowly to minimize damage to the cells. While the size of the syringe and needle are by no means limiting, a large gauge needle is preferred for aspiration. This facilitates the transfer and reduces cell damage.

Application of the activated leukocyte composition to the ulcer comprises injecting the composition into the wound. The entire sample in the syringe can be deployed and the clinician can choose to administer additional ALC if it is determined to be necessary based on clinical parameters.

The 18G needle used for aspiration is exchanged with a needle ranging in size from 22-35G. The ALC may be injected into the wound in various locations. Injection can occur about every one centimeter to about every three centimeters for the entire length of the wound. At each injection site, 0.1-0.3 ml of ALC is injected.

The entire syringe can be injected at one time into a single site within the wound.

Aspect(s) of the present invention will now be described in accordance with the following non-limiting examples.

Example 1 - Analysis of Cellular Activation.

An activated leukocyte composition made in accordance with the preferred embodiment of the present invention was quantified by the analyis of various cell surface markers. An increase in platelet aggregation with either monocytes or granulocytes is a sign of activation of the monocytes and granulocytes through the expression of P selectin. CD62L is a plasma membrane protein which is shed during activation and thus decreases with cell activation. CD42b is a platelet activation marker involved in the process of coagulation as an aggregating factor. It interacts with extra-cellular matrix as well as with adhesion molecules and also used in the present invention as an indicator of monocyte and granulocyte activation.

Cells were sampled at three time points: fresh buffy coat (Fr.BC); incubated buffy coat (IBC); and final activated leukocyte composition (FP) (Table 1).

**Table 1: Cell surface markers indicating leukocyte activation**

| % of CD62L positive cells | | | |
|---|---|---|---|
| | Fr.BC | IBC | FP |
| Monocytes | 68 | 55 | 44 |
| Granulocytes | 97 | 47 | 39 |

| % of CD42b positive cells | | | |
|---|---|---|---|
| Monocytes | 26 | 74 | 92 |
| Granulocytes | 3 | 15 | 39 |

Fr.BC was taken immediately after the preparation of Buffy Coat. IBC was taken after the first incubation period and FP was taken from the Final Product. At each time point, cells were labeled with specific monoclonal antibodies (allophycocyanin (APC) conjugated anti-CD14, phycoerythrin(PE) conjugated anti-CD42b, and fluorescein-isothiocyanate (FITC) conjugated anti-CD62L antibodies), and then analyzed by FACS.

Cells from each time point were washed with FACS staining solution (PBS, 2% Normal Mouse Serum; 0.02% Sodium Azide), aliquoted and stained according to staining protocol. Briefly, 0.5x(10⁶/µl) cells were incubated with appropriate monoclonal antibodies for 30 min. at room temperature (RT) or at +4°C in the dark. After incubation, the cell composition was treated with erythrocyte lysis buffer, washed, and then finally re-susupended in PBS for FACS acquisition. For CD62L positive (+) staining, the cell composition was incubated with anti-human CD14 (APC) and anti-human CD62L (FITC) antibodies at +4°C. For CD42b+ staining, the cell composition was incubated with anti-human-CD14 (APC) and anti-human CD42b (PE) at room temperature (RT). Negative control cell composition was incubated with anti-human-CD14 and appropriate isotype controls under the relevant conditions. Cell-associated light scatter and fluorescence were analyzed using a FACS CALIBUR (Becton Dickinson). Monocytes were determined as CD14 positive cells and granulocytes were identified by their characteristic light scatter properties. The CD62L or CD42b positive cells were defined as the percentage of monocytes and granulocytes with fluorescence greater than a threshold, as determined by monocytes and granulocytes incubated with appropriate isotype control antibody.

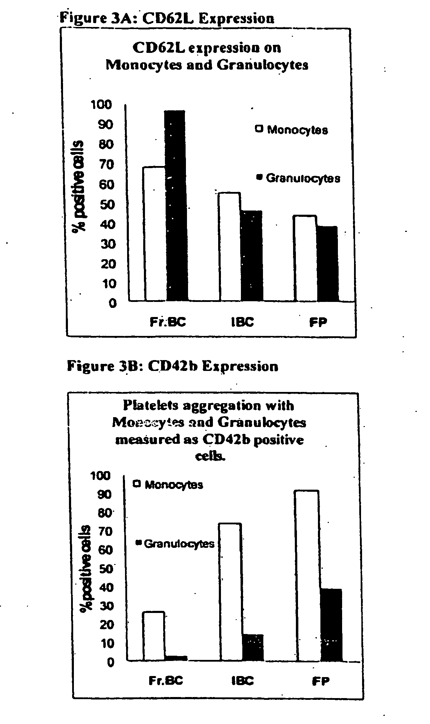

As shown in Figures 3A and 3B and Table 1, the results were consistent with the expectation that CD62L expression would decrease as activation progressed and CD42b would increase as activation progressed. These data demonstrate that leukocytes became activated.

### EXAMPLE 2-ANALYSIS OF AN ACTIVATED LEUKOCYTE COMPOSITION

Tables 2 and 3 depict the cellular compositions of the final ALC as determined by analysis with a Cell Dyn analyzer. Cells were analyzed after re-suspending the activated leukocytes in serum. Viable cells were stained using trypan blue exclusion and observed under a microscope.

**Table 2: Composition of Activated Leukocyte Composition**

| | Platelets (10³/µl) | Erythrocytes (10⁶/µl) | Leukocytes (10³/µl) |
|---|---|---|---|
| Concentration in final ALC | 46.8 | 0.1 | 6.8 |
| Standard Deviation | 39.2 | 0.06 | 3.8 |

**Table 3: Leukocyte Composition in ALC**

| **Leukocytes** | | | | | |
|---|---|---|---|---|---|
| | **Granulocytes** | | | | |
| | Neutrophills % | Basophilis % | Eosinophilsophills% | Monocytes % | Lymphocytes % |
| % in final ALC | 65.5 | 1.6 | 4.6 | 9.1 | 18.5 |
| Standard Deviation | 8.2 | 0.3 | 3 | 2.1 | 4.1 |
| Range | 52-78 | 1-2 | 1-9 | 6-12 | 13-24 |

The Cell Dyn results, as shown in Table 2, show the ALC contained platelets in the amount of 46.8+/- 39.2 (103/µl), erythrocytes in the amount of 0.1 +/-.03(106/µl) and leukocytes in the amount of 6.8 +/- 3.8(103/µl). Based on the Cell Dyn analysis, it was also determined that the leukocyte composition of the ALC (as shown in Table 3) contained 52%-78% neutrophils, 1-2% basophils, 1-9% eosinophils, 6%-12% monocytes, and 13%-24% lymphocytes, taking into account standard deviation. The ranges depicted in these tables represent the high and low results after 8 separate analyses using the Cell Dyn analyzer.

Example 3

Comparison Between Inventive Method And Prior Art Process

To highlight the various unexpected advantages associated with the present invention, embodiments thereof were compared to the process disclosed in U.S. Patent 6, 146, 890, to Danon ("*Danon*").

*Inventive Embodiments*

Referring to Figs. 1 and 2, immediately after separating whole blood into its primary components, i.e., red blood cells, plasma, and Buffy coat, the Buffy coat was transferred from Bag C to Bag 4 and incubated for 12 hours ±2 hours at room temperature. This step was followed by transferring the distilled water from Bag 1 to Bag 4 (or bag 5), for purposes of conducting hypo-osmotic shock treatment (resulting in the production of a hemolysate). This treatment was conducted for approximately 45 seconds. Immediately thereafter, the buffered sodium chloride solution contained in Bag 2 was transferred to Bag 4 (or bag 5) for purposes of restoring isotonicity to the Buffy coat (leukocytes).

Contemporaneous with the incubation of the Buffy coat, above, the buffered calcium chloride solution in Bag 3 was transferred to Bag B containing the plasma portion, for purposes of allowing for coagulation of the plasma. This was allowed to take place over the course of 12 hours ±2 hours, plus the additional short time during which the leukocytes were subjected to hypo-osmotic shock and then restoration of isotonicity.

Immediately following restoration of isotonicity, the entire bag assembly was subjected to centrifugation (typically about 8 to about 10 minutes), followed by separation of the cells from the hemolysate. In so doing, the cells were exposed to hemolysate influence for a minimal period of time, i.e., approximately 10 minutes. Following centrifugation, the supernatant of the hemolysate in Bag 1 (or bag 5) was discarded, and fresh medium was added to Bag 1 (or bag 5), followed by incubation for about 1-2 hours at 37°C. Following incubation, the cells were subjected to a single wash step.

*Comparative (Prior Art) Procedure*

Following the teachings in *Danon,* the Buffy coat was subjected to hypo-osmotic shock immediately after processing the whole blood and separating it into its 3 main components, i.e., red blood cells, plasma, and the Buffy coat. Thus, in sharp contrast to the present invention, *Danon's* procedure does not entail incubation of the Buffy coat after separation of the main blood components and prior to subjecting the Buffy coat to hypo-osmotic shock. The hypo-osmotic shock was conducted while the Buffy coat was contained in Bag PB₃.

As a separate step, and after hypo-osmotic shock the buffered calcium chloride solution was transferred from Bag PB₆ to Bag PB₂ which contains the plasma, followed by deep-freezing the plasma in PB₂ for 10 minutes, and then placing it in a water bath at 37°C for an additional 30 minute incubation period. During this time, the Buffy coat fraction which had been subjected to hypo-osmotic shock was allowed to stand wherein the leukocytes remained exposed to the hemolysate.

Following the conclusion of this coagulation process, which in total lasted about 40 minutes, the entire bag system was subject to centrifugation, and the hemolysate was discarded. Thus, the cells were exposed to the hemolysate for a period of at least about 55 minutes (i.e., which included the 40-minute standing period that coincided with the plasma coagulation, and an additional 15 minutes for centrifugation). In contrast, in the inventive method, the leukocytes were centrifuged immediately for about 5 min and thus were exposed to hemolysate for much less time, i.e., less than 10 minutes.

After the supernatant from PB₃ was discarded, fresh medium was added thereto, followed by transferring the entire suspension back to PB₇, which was then incubated for about 17 hours at 37°C. Following incubation, the cells were washed 3 times. In contrast, in the embodiments of the present invention, the incubation period was conducted in coagulated plasma for 1-2 hours, and washed only one time.

Until the aforesaid incubation was conducted, all prior steps were carried out in transfusion bags, which were the bags the whole blood was collected in. In contrast, the inventive embodiments entailed use of infusion bags which are usually used to administer intravenous solutions such as saline.

Danon's procedure was conducted 4 times and compared to the results generated by the embodiment of the present invention. The results, as set forth below, were averaged.

*RESULTS*

The total amount of the white blood cells (leukocytes) obtained from a standard blood unit, calculated as a final concentration multiplied by final volume of cell suspension, was determined for each batch of whole blood. The averaged results for the 111 batches of whole blood processed according to the method of the present invention, and the 4 batches that were processed in accordance with the procedure disclosed in *Danon,* are set forth in Table 4.

**Table 4. Yield of WBC per blood unit - (The present invention yielded ~90-fold higher amounts of WBCs.)**

| Number of batches | WBC, (x10⁶) | |
|---|---|---|
| | Average | SD |
| Present invention (n=111) | 449 | 220 |
| *Danon* (n=4) | 5 | 4 |

The results show that the method of the present invention resulted in about 90 times greater total number of leukocytes obtained from a standard blood unit (about 450 ml), as compared to *Danon's* procedure. More generally, the present method results in a yield of at least about 100 x 10⁶, 125 x 10⁶, 150 x 10⁶, 175 x 10⁶, 200 x 10⁶, 225 x 10⁶, 250 x 10⁶, 275 x 10⁶, 300 x 10⁶, 325 x 10⁶, 350 x 10⁶, 375 x 10⁶, 400 x 10⁶, 425 x 10⁶, 450 x 10⁶, 475 x 10⁶, 500 x 10⁶, or higher leukocytes per standard blood unit (including all sub-ranges thereof).

The number of viable cells contained within the total cell population, expressed as a percentage, was measured by the Trypan Blue exclusion method. The cells were counted in a Newbauer hamemocytometer after suspension in Trypan Blue (1:1 ratio) for evaluation of cell count and percentage of viability. The results are presented in Table 5.

**Table 5.**

| | Viability (% live cells) | |
|---|---|---|
| Procedure | Average | SD |
| Present invention (n=111) | 98% | 0.02% |
| *Danon* (n=4) | 77% | 6% |

The data in Table 5 show that in addition to the lower yield (as shown in Table 4), *Danon's* procedure resulted in cell suspension with significantly lower viability. That is, almost a quarter (*i.e*., 23%) of the preparation was composed of dead leukocytes (greater than a 10-fold higher percentage of dead cells),)). In sharp contrast, almost all of the white blood cells processed according to the inventive method were determined to be viable. More generally, the inventive ALC may contain at least 80%, 81%, 82%, 83%, 84%, 85%, 86%, 87%, 88%, 89%, 90%, 91%, 92%, 93%, 94%, 95%, 96%, 97%, 98% or greater, viable leukocytes based on this total number of leukocyte cells in the ALC (including all sub-ranges thereof).

Expression of the CD11b activation marker on granulocytes was measured by flow cytometry, and presented as a percentage of CD11b positive cells in granulocyte population (CD15 positive cells). Cells sampled from final product were co-stained with anti-CD11b conjugated to fluorescein isothiocynate (FITC) and anti-CD15 conjugated to phycoerythrin (PE), and analyzed using FACSCalibur flow cytometer (Becton Dickinson Immunocytometry Systems, San Jose, CA, USA). CD11b expression on granulocytes in cells from 81 final product batches performed in accordance with the inventive method, and from 3 final product batches produced according *Danon's* procedure, were analyzed.

**Table 6 CD11b expression on Granulocytes**

| | % of Granulocytes expressing CD11b activation marker | |
|---|---|---|
| Procedure | Average | SD |
| Present invention (n=81) | 84.6 | 6.2 |
| Danon (n=3) | 46.9 | 9.2 |

As shown by the data contained in Table 6, the inventive method yielded almost two-fold higher activated granulocytes on a percentage basis as compared to *Danon's* procedure. More generally, the ALCs of the present invention may contain at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, or higher, CD11b(+) granulocytes, relative to the total granulocyte population in the ALC (including all sub-ranges thereof).

The results of the comparative experimentation demonstrate that the presently disclosed invention achieves at least 3 unexpected results as compared to the process disclosed in *Danon,* namely, a greater yield of *viable* leukocytes, a high percentage of viable cells, and a higher level of granulocyte activation, as shown by higher expression of the CD11b activation marker. These increases are dramatic and unexpected.

List of Publications cited in Application

J. Li, et al., Pathophysiology of Acute Wound Healing. Clinical Dermatol. 2007 Jan-Feb; 25(1):9-18.

G. Broughton 2nd, et al., Wound Healing: An Overview. Plast Reconstr Surg. 2006 Jun; 117(7 Suppl):1e-S-32e-S.

AK Tsirogianni, et al., Wound Healing: Immunological Aspects. Injury. 2006 Apr; 37 Suppl 1:S5-12. Epub 2006

AJ Singer, et al., Cutaneous Wound Healing. New Eng. J. Med. 1999; 341(10):738-46.

P. Martin, Wound Healing--Aiming For Perfect Skin Regeneration. Science 1997; 276(5309):75-81.

AD Agaiby, et al., Immuno-Inflammatory Cell Dynamics During Cutaneous Wound Healing. J. Anat. 1999 Nov; 195 (Pt 4):531-42.

DWH Riches, Macrophage Involvement In Wound Repair, Remodeling and ®brosis. In The Molecular and Cellular Biology of Wound Repair, (1996) lnd edn (ed. Clark RAF), pp. 95±141. New York, London: Plenum Press.

D. Greiling, et al., Fibronectin Provides a Conduit for Fibroblast Transmigration from Collagenous Stroma into Fibrin Clot Provisional Matrix. J. Cell. Sci. 1997;110:861-70

MG Tonnesen, et al., Angiogenesis In Wound Healing. J. Investig. Dermatol. Symp. Proc. 2000; 5(1):40-6.

JP Kim, et al., Mechanisms of Human Keratinocyte Migration on Fibronectin: Unique Role of RGD Site and Integrins. J. Cell. Physiol. 1992; 151:443-50.

JJ Tomasek et al., Myofibroblasts and Mechano-Regulation of Connective Tissue Remodelling. Nat. Rev. Mol. Cell. Biol. (2002) 3:349-363

S Werner, et al., Regulation of Wound Healing by Growth Factors and Cytokines. Physiol Rev. 2003 Jul; 83 (3) :835-70.

D.A. Keen, Review of Research Examining the Regulatory Role of Lymphocytes in Normal Wound Healing. J. Wound Care. 2008

J Jameson, et al., A Role for Skin - T Cells in Wound Repair. Science 296: 747-749, 1992.

WL Havran, et al., Epithelial Cells and Their Neighbors. III. Interactions Between Intraepithelial Lymphocytes and Neighboring Epithelial Cells. Am. J. Physiol. Gastrointest Liver Physiol. 2005 Oct; 289(4):G627-30

J Parkin, et al., An Overview of the Immune System. Lancet. 2001; 357:1777-1789

A Moretta, et al., Human NK Cells: From HLA Class ISpecific Killer Ig-like Receptors to the Therapy of Acute Leukemias. Immunol. Rev. 2008 Aug; 224:58-69.

BM Doshi, et al., Wound Healing From a Cellular Stress Response Perspective. Cell Stress Chaperones. 2008 Dec; 13(4):393-9.

GJ Moran, et al., Antimicrobial Prophylaxis for Wounds and Procedures in the Emergency Department. Infect. Dis. Clin. North Am. 2008

MA Fonder, et al., Treating the Chronic Wound: A Practical Approach to the Care of Nonhealing Wounds and Wound Care Dressings. J. Am. Acad. Dermatol. 2008 Feb; 58(2):185-206.

JA Thackham, et al., The Use of Hyperbaric Oxygen Therapy to Treat Chronic Wounds: A Review. Wound Repair Regen. 2008, 1998 May-Jun;16(3):321-30

DR Berlowitz, et al., Are all pressure ulcers the result of deep tissue injury? A Review of the Literature. Ostomy Wound Manage. 2007 Oct; 53(10):34-8.

K. Girouard, et al., The symptom of pain with pressure ulcers: a review of the literature. Ostomy Wound Manage. 2008 May; 54(5):30-40, 32.

All publications cited in the specification, including both patent publications and non-patent publications, are indicative of the level of skill of those skilled in the art to which this invention pertains.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. A method for making an activated leukocyte composition comprising:
a. incubating human leukocytes at a
(i) temperature of 12°C - 28°C for a time ranging from about 90 minutes upwards of about 2, 3, 4, 5, 6, 7, 8, or 12 to 20 hours or
(ii) temperature of 12 °C - 37°C for a time ranging from 5-24 hours so that the leukocytes transition from a quiescent to a functionally active state;
b. subjecting the leukocytes to hypo-osmotic shock; and
c. adding to the leukocytes of step b, a physiologically acceptable salt solution in an amount effective to restore isotonicity.

2. A method of claim 1 further comprising:
contacting a separate sample of human plasma with a coagulating agent to obtain serum, wherein the leukocytes and the plasma may be obtained from the same or different donors; and
mixing the leukocytes of step c with the serum of step d.

3. The method of any of claim 1 or 2, wherein the leukocytes are obtained from a donor having an O negative blood type and the plasma is obtained from a donor having an AB positive blood type.

4. The method of any one of claims 1-3, wherein the hypo-osmotic shock comprises contacting the leukocytes with water, and wherein the physiologically acceptable salt solution is a 0.9% sodium chloride solution.

5. The method of any one of claims 1-4, further comprising incubating the leukocytes in the serum at 37°C.

6. An activated leukocyte composition prepared by the method of claim 1, wherein the composition comprises:
a) 40% - 90% granulocytes;
b) 5% - 20% monocytes; and
c) 5% - 30% lymphocytes.

7. The composition of claim 6, wherein the granulocytes comprise:
a) 52% - 78% neutrophils;
b) 1% - 9% eosinophils; and
c) 1% - 2% basophils.

8. The composition of claim 6 or 7, wherein the lymphocytes comprise:
a) 7% - 25% B cells (CD19+);
b) 20% - 30% NK cells (CD3-/CD56+);
c) 40% - 60 T cells (CD3+);
d) 0% - 30 of NKT cells (CD3+/CD56+);
e) 8% - 20% of T helper cells, (CD4+/CD3+); and
f) 20% - 30% of CD8+/CD3+ cells.

9. The composition of any one of claims 6-8 for use as a medicament.

10. The composition of any one of claims 6-8 for use as a wound healing agent.

11. The compositions of any of claims 6-8 for use in treating a wound.

12. The composition for use according to claim 11, wherein the wound is a decubital ulcer, a pressure ulcer, a lower extremity ulcer in a diabetic patient, a deep sternal wound, a post-operative wound, a refractory post-operative wound of the trunk area, a wound to the great saphenous vein after coronary revascularization and harvesting of the great saphenous vein, a wound caused by trauma, an anal fissure, or a venous ulcer.

13. A dressing comprising a composition of any one of claims 6-8.

14. A physiologically inert and/or resorbable matrix or scaffold comprising a composition of any one of claims 6-8.

## Patentansprüche

1. Verfahren zum Erstellen einer aktivierten Leukozytenzusammensetzung, das umfasst:
a. Inkubieren humaner Leukozyten bei einer
(i) Temperatur von 12°C bis 28°C über einen Zeitraum im Bereich von etwa 90 Minuten bis zu etwa 2, 3, 4, 5, 6, 7, 8 oder 12 bis 20 Stunden oder
(ii) Temperatur von 12°C bis 37°C über einen Zeitraum im Bereich von 5 bis 24 Stunden,
so dass die Leukozyten von einem ruhenden zu einem funktional aktiven Zustand übergehen;
b. Aussetzen der Leukozyten gegenüber einem hypoosmotischen Schock; und
c. Hinzufügen einer physiologisch akzeptablen Salzlösung in einer zur Wiederherstellung der Isotonizität wirksamen Menge zu den Leukozyten von Schritt b.

2. Verfahren nach Anspruch 1, das weiterhin umfasst:
das In-Kontakt-Bringen einer separaten Probe von humanem Plasma mit einem Gerinnungsmittel, um Serum zu erhalten, wobei die Leukozyten und das Plasma von dem gleichen Spender oder verschiedenen Spendern gewonnen werden können; und
das Mischen der Leukozyten von Schritt c mit dem Serum von Schritt d.

3. Verfahren nach Anspruch 1 oder 2, wobei die Leukozyten von einem Spender mit der Blutgruppe O negativ gewonnen werden und das Plasma von einem Spender mit der Blutgruppe AB positiv gewonnen wird.

4. Verfahren nach einem der Ansprüche 1 bis 3, wobei der hypoosmotische Schock das In-Kontakt-Bringen der Leukozyten mit Wasser umfasst und wobei die physiologisch akzeptable Salzlösung eine 0,9%ige Natriumchloridlösung ist.

5. Verfahren nach einem der Ansprüche 1 bis 4, das weiterhin das Inkubieren der Leukozyten in dem Serum bei 37°C umfasst.

6. Aktivierte Leukozytenzusammensetzung, die anhand des Verfahrens nach Anspruch 1 hergestellt wird, wobei die Zusammensetzung umfasst:
a) 40% - 90% Granulozyten;
b) 5% - 20% Monozyten; und
c) 5% - 30% Lymphozyten.

7. Zusammensetzung nach Anspruch 6, wobei die Granulozyten umfassen:
a) 52% - 78% Neutrophile;
b) 1% - 9% Eosinophile; und
c) 1% - 2% Basophile.

8. Zusammensetzung nach Anspruch 6 oder 7, wobei die Lymphozyten umfassen:
a) 7% - 25% B-Zellen (CD19+);
b) 20% - 30% NK-Zellen (CD3-/CD56+);
c) 40% - 60 T-Zellen (CD3+);
d) 0% - 30 NKT-Zellen (CD3+/CD56+);
e) 8% - 20% T-Helferzellen (CD4+/CD3+); und
f) 20% - 30% CD8+/CD3+-Zellen.

9. Zusammensetzung nach einem der Ansprüche 6 bis 8 zur Verwendung als Medikament.

10. Zusammensetzung nach einem der Ansprüche 6 bis 8 zur Verwendung als Wundheilungsmittel.

11. Zusammensetzungen nach einem der Ansprüche 6 bis 8 zur Verwendung beim Behandeln einer Wunde.

12. Zusammensetzung zur Verwendung nach Anspruch 11, wobei die Wunde ein Dekubitusgeschwür, ein Druckgeschwür, ein Geschwür der unteren Gliedmaßen bei einem diabetischen Patienten, eine tiefe sternale Wunde, eine postoperative Wunde, eine refraktäre postoperative Wunde des Rumpfbereichs, eine Wunde der großen Saphenavene nach koronarer Revaskularisation und Ernten der großen Saphenavene, eine durch ein Trauma verursachte Wunde, eine Analfissur oder ein venöses Geschwür ist.

13. Verband, der eine Zusammensetzung nach einem der Ansprüche 6 bis 8 umfasst.

14. Physiologisch träge und/oder resorbierbare Matrix oder Gerüst, das eine Zusammensetzung nach einem der Ansprüche 6 bis 8 umfasst.

## Revendications

1. Procédé de fabrication d'une composition de leucocytes activés, comprenant les étapes consistant à :
a. incuber des leucocytes humains
(i) à une température de 12 °C à 28 °C pendant une période de temps allant d'environ 90 minutes ou plus jusqu' à environ 2, 3, 4, 5, 6, 7, 8 ou 12 à 20 heures ou
(ii) à une température de 12 °C à 37 °C pendant une période de temps allant de 5 à 24 heures
de sorte que les leucocytes passent d'un état quiescent à un état fonctionnellement actif ;
b. soumettre les leucocytes à un choc hypo-osmotique ; et
c. ajouter aux leucocytes de l'étape b une solution de sel acceptable au plan physiologique en quantité efficace pour restaurer l'isotonicité.

2. Procédé selon la revendication 1, comprenant en outre les étapes consistant à :
mettre en contact un échantillon séparé de plasma humain avec un agent de coagulation pour obtenir du sérum, dans lequel les leucocytes et le plasma peuvent être tirés des mêmes donneurs ou de donneurs différents ; et
mélanger éventuellement les leucocytes de l'étape c au sérum de l'étape d.

3. Procédé selon l'une quelconque des revendications 1 ou 2, dans lequel les leucocytes sont tirés d'un donneur ayant un type de sang O négatif et le plasma est tiré d'un donneur ayant un type de sang AB positif.

4. Procédé selon l'une quelconque des revendications 1 à 3, dans lequel le choc hypo-osmotique comprend la mise en contact des leucocytes avec de l'eau et dans lequel la solution de sel physiologiquement acceptable est une solution à 0,9 % de chlorure de sodium.

5. Procédé selon l'une quelconque des revendications 1 à 4, comprenant par ailleurs l'incubation des leucocytes dans le sérum à 37 °C.

6. Composition de leucocytes activés préparée par le procédé de la revendication 1, dans laquelle la composition comprend :
a) 40 % à 90 % de granulocytes ;
b) 5 % à 20 % de monocytes ; et
c) 5 % à 30 % de lymphocytes.

7. Composition selon la revendication 6, dans laquelle les granulocytes comprennent :
a) 52 % à 78 % de neutrophiles ;
b) 1 % à 9 % d'éosinophiles ; et
c) 1 % à 2 % de basophiles.

8. Composition selon la revendication 6 ou la revendication 7, dans laquelle les lymphocytes comprennent :
a) 7 % à 25 % de cellules B (CD19+) ;
b) 20 % à 30 % de cellules NK (CD3-/CD56+) ;
c) 40 % à 60 % de cellules T (CD3+) ;
d) 0 % à 30 % de cellules NKT (CD3+/CD56+) ;
e) 8 % à 20 % de cellules T auxiliaires (CD4+/CD3+) ; et
f) 20 % à 30 % de cellules CD8+/CD3+.

9. Composition selon l'une quelconque des revendications 6 à 8 pour usage comme médicament.

10. Composition selon l'une quelconque des revendications 6 à 8 pour usage comme agent de cicatrisation d'une plaie.

11. Composition selon l'une quelconque des revendications 6 à 8 pour utilisation dans le traitement d'une blessure.

12. Composition pour utilisation selon la revendication 11, dans lequel la blessure est un ulcère du décubital, un ulcère de pression, un ulcère d'une extrémité inférieure chez un patient diabétique, une blessure profonde au sternum, une blessure post-opératoire, une blessure post-opératoire réfractaire de la zone du tronc, une blessure à la grande veine saphène après revascularisation coronaire et prélèvement de la grande veine saphène, une blessure provoquée par un traumatisme, une fissure anale ou un ulcère veineux.

13. Pansement comprenant une composition selon l'une quelconque des revendications 6 à 8.

14. Matrice ou charpente inerte au plan physiologique et/ou résorbable comprenant une composition selon l'une quelconque des revendications 6 à 8.
